# EUROPEAN PATENT APPLICATION

(11) **EP 1 369 687 A1**
(43) Date of publication of application: **10.12.2003**
(21) Application number: 03253470.3
(22) Date of filing: 03.06.2003
(51) Int. Cl.: G01N 33/487, C12Q 1/00

(54) **Biosensor**

(30) Priority: 03.06.2002 JP 2002161740
(71) Applicant: MATSUSHITA ELECTRIC INDUSTRIAL CO., LTD., Kadoma-shi, Osaka 571-8501 (JP)
(72) Inventor: Miyamoto,Yoshiko, Suita-shi, Osaka 565-0821 (JP); Yamamoto, Tomohiro, Hirakata-shi, Osaka 573-0018 (JP); Hasegawa, Miwa, Ako-gun, Hyogo 678-1205 (JP); Yoshioka, Toshihiko, Hirakata-shi, Osaka 573-0035 (JP)
(74) Representative: Bradley, Josephine Mary

(57) **Abstract**

In a biosensor comprising an electrically insulating base plate, an electrode system including a working electrode and a counter electrode formed on the base plate, and a reaction layer formed on or in the vicinity of the electrode system, at least a surface of the reaction layer is made porous, so as to provide a biosensor of good response characteristic in which a reaction layer containing an enzyme dissolves quickly into a small amount of a sample solution and the enzyme reaction is effectively utilized.

In one embodiment the reaction layer contains the enzyme and an aggregate of fine particles having an average diameter of between 0.1 µm and 1 µm.

The fine particles may be made of a material selected from a polymer compound, ceramic, glass, diamond and carbon.

## Description

### BACKGROUND OF THE INVENTION

The present invention relates to a biosensor for determining the quantity of a substrate (specific component) contained in a sample, e.g., biological samples such as blood and urea, materials and products in food industries and fruit juice, in a highly accurate, speedy and easy manner.

There has been provided a biosensor capable of easily determining the quantity of a specific component (substrate) in biological samples and foods without diluting or stirring the sample solution. For example, Japanese Laid-Open Patent Publication No. HEI 3-202764 discloses a biosensor including an electrode system formed on an electrically insulating base plate by screen printing and a reaction layer formed on the electrode system. The reaction layer contains oxidoreductase and an electron mediator (electron acceptor).

This biosensor determines the substrate concentration in a sample in the following manner.

First, a sample solution is dropped onto the reaction layer of the biosensor. Then, the reaction layer dissolves to cause an enzyme reaction between the substrate in the sample solution and oxidoreductase in the reaction layer. Subsequently to the enzyme reaction, the electron mediator is reduced. After a predetermined time, a voltage is applied to the electrode of the sensor to electrochemically oxidize the reduced electron mediator. At that time, an oxidation current is obtained, from which the substrate concentration in the sample solution is determined.

However, since the reaction layer of the prior art biosensor is formed by a method of dropping and drying a solution containing a water-soluble component, it is difficult to form the reaction layer into a thin film. Further, it is also difficult to dry the reaction layer uniformly throughout its thickness and the performance of the sensor may unfavorably vary depending on the dry state of the surface of the reaction layer. Accordingly, the enzyme needs to be contained in an excessive amount in the reaction layer as compared with the amount of the substrate contained in the sample. Further, since the reagent is contained in a large amount in the reaction layer, it takes long time to dissolve the reaction layer.

Japanese Laid-Open Patent Publication No. 2001-208716 discloses a sensor strip comprising an electrode support, an electrode set formed thereon and microscopic grains. In this sensor, the microscopic grains are used to reduce the amount of the sample required for the measurement, thereby increasing the dissolution rate of the reagent.

### BRIEF SUMMARY OF THE INVENTION

In view of the-above described problems concerning the prior art, an object of the present invention is to provide a biosensor of good response characteristic in which the reaction layer containing the enzyme dissolves rapidly into a small amount of a sample solution and the enzyme reaction is effectively utilized.

Another object of the present invention is to provide a biosensor in which the above-described reaction layer is easily formed.

To achieve the above-described objects, the present invention provides a biosensor comprising an electrically insulating base plate, an electrode system including a working electrode and a counter electrode formed on the base plate, and a reaction layer formed on or in the vicinity of the electrode system, at least a surface of the reaction layer being porous.

To make the reaction layer porous, preferably
the reaction layer contains at least an enzyme and an aggregate of fine particles having an average diameter of not smaller than 0.1 µm and not larger than 1 µm.

The reaction layer may entirely be porous.

The porosity of the reaction layer is preferably 50% or more and the upper limit thereof may be about 90%.

Preferably the fine particles are made of a material selected from the group consisting of a polymer compound, ceramic, glass, diamond and carbon.

Preferably the reaction layer further contains an electron mediator.

A layer containing the electron mediator may be formed in the vicinity of the reaction layer.

In a preferred embodiment of the present invention the reaction layer contains cholesterol esterase, a surfactant and at least one of cholesterol oxidase and cholesterol dehydrogenase and a layer containing the electron mediator is formed in the vicinity of the reaction layer.

While the novel features of the invention are set forth particularly in the appended claims, the invention, both as to organization and content, will be better understood and appreciated, along with other objects and features thereof, from the following detailed description taken in conjunction with the drawings.

### BRIEF DESCRIPTION OF THE SEVERAL VIEWS OF THE DRAWING

FIG. 1 is a perspective view of a disassembled biosensor according to an example of the present invention, in which a reaction layer is omitted.

FIG. 2 is a schematic vertical section of the above biosensor.

FIG. 3 is a graph illustrating comparison among response characteristics of sensors according to Examples 1 and 2 and Comparative Example 1.

FIG. 4 is an optical photomicrograph of the reaction layer formed in Example 1.

FIG. 5 is a graph illustrating comparison between response characteristics of sensors of Example 5 and Comparative Example 3.

### DETAILED DESCRIPTION OF THE INVENTION

The biosensor of the present invention comprises an electrically insulating base plate, an electrode system including a working electrode and a counter electrode formed on the base plate, and a reaction layer formed on or in the vicinity of the electrode system. The biosensor is characterized in that at least a surface of the reaction layer is porous.

To make the reaction layer porous, preferably the reaction layer contains at least an enzyme and an aggregate of fine particles having an average diameter of not smaller than 0.1 µm and not larger than 1 µm.

The reaction layer may entirely be porous.

The fine particles used herein are preferably made of a material selected from the group consisting of a polymer compound, ceramic such as silica, alumina and titanium oxide, glass, diamond and carbon.

In the present invention, the reaction layer having at least a porous surface is preferably formed by dispersing the fine particles in a solution for forming the reaction layer, preferably an aqueous solution containing an enzyme, spreading the resulting solution on or in the vicinity of the electrode system on the base plate and drying the solution.

If the average diameter of the fine particles is 1 µ m or more, the fine particles do not disperse smoothly in the solution for forming the reaction layer, which requires constant stirring. Thereby, the production process is complicated. Further, if the solution in which the fine particles are dispersed inhomogeneously is used, the resulting reaction layer becomes inhomogeneous and the porous reaction layer cannot be obtained. In such a case, the resulting reaction layer does not dissolve quickly on a whole in use of the biosensor, which deteriorates the response characteristic.

On the other hand, if the average diameter of the fine particles is smaller than 0.1 µm, voids in the fine particles in the resulting reaction layer are narrowed, the porosity of the reaction layer decreases and the surface area of the enzyme does not increase.

Considering above, the present invention utilizes the fine particles having the average diameter of not smaller than 0.1 µm and not larger than 1 µm. Preferably the fine particles have an average diameter of greater than or equal to 0.1 µm and less than 1 µm. Thereby, fine voids are formed in the reaction layer and hence the reaction layer becomes porous. This allows the sample solution supplied to the sensor to permeate rapidly into the reaction layer while dissolving the enzyme. As a result, the reaction layer quickly dissolves into the sample solution and the enzyme reaction proceeds speedily. Thus, highly accurate measurement is realized without causing variations in response characteristics.

The amount of the fine particles contained in the reaction layer is suitably 10 to 99 % by volume ratio with respect to the reaction layer with a view to forming a thin enzyme layer on the fine particle surface. Thereby, the porous reaction layer which dissolves easily into the sample solution is formed. More preferably, the volume ratio is 10 to 20 %.

Examples of the enzyme used in the present invention include glucose oxidase, glucose dehydrogenase, lactate oxygenase, lactate dehydrogenase, fructose oxidase, cholesterol oxidase, cholesterol dehydrogenase, cholesterol esterase, mutarotase, invertase, ascorbate oxidase, alcohol oxidase and the like. Among them, one or a combination of two or more may be used.

The reaction layer of the biosensor according to the present invention may contain an electron mediator as described above. Examples of the electron mediator include ferricyanide and salts thereof, p-benzoquinone and derivatives thereof, phenazine methosulfate, methylene blue, ferrocene and derivatives thereof and the like. Among them, one or a combination of two or more may be used as the electron mediator. If the electron mediator is not contained in the reaction layer, the quantity of the substrate can be determined by using an enzyme or hydrogen peroxide in the sample solution.

If the electron mediator is mixed with the enzyme to form the reaction layer, the activity of the enzyme may be deteriorated by the electron mediator during storage. Accordingly, it is preferred that the electron mediator is contained in a layer isolated from the enzyme. In the case of a cholesterol sensor, are used a surfactant, cholesterol esterase for converting cholesterol ester into free cholesterol, and at least one of cholesterol oxidase and cholesterol dehydrogenase. In such a sensor, the electron mediator is preferably contained in a layer isolated from the enzymes.

A solvent for dissolving therein the enzyme and/or the electron mediator to prepare a solution for forming the reaction layer may be selected from water, various aqueous solutions such as phosphoric acid buffer, Tris-HCl buffer, phosphoric acid buffer, acetic acid buffer and sodium chloride aqueous solution and organic solvents such as methanol, ethanol, toluene and acetone. The solvent is preferably one of them or a mixture solution of two or more of them. The content of the fine particles to be added to the solution for forming the reaction layer is preferably in the range of 5 to 20 % in terms of volume concentration.

The reaction layer of the biosensor according to the present invention may contain a hydrophilic polymer in addition to the enzymes and the electron mediator. The addition of the hydrophilic polymer to the reaction layer prevents the reaction layer from peeling off the base plate or the surface of the electrode system. Further, the fracture of the reaction layer surface is also prevented, which is effective in enhancing the reliability of the biosensor. The hydrophilic polymer may cover the electrode system, causing the same effect as the above.

Examples of the hydrophilic polymer include carboxymethyl cellulose, hydroxyethyl cellulose, hydroxypropyl cellulose, methyl cellulose, ethyl cellulose, ethyl hydroxyethyl cellulose, carboxy methyl ethyl cellulose, polyvinylpyrrolidone, polyvinyl alcohol, polyamino acid such as polylysine, polystyrene sulfonate, gelatin and derivatives thereof, polymers of acrylic acid or derivatives thereof, polymers of maleic anhydride or salts thereof and starch or derivatives thereof. Among them, carboxymethyl cellulose is particularly preferred.

An oxidation current may be measured by a measurement method on a two-electrode system using only a measuring electrode and a counter electrode or a three-electrode system using a reference electrode in addition, among which the three-electrode system allows measurement with greater accuracy.

Hereinafter, a preferred embodiment of the biosensor according to the present invention is described with reference to the figures.

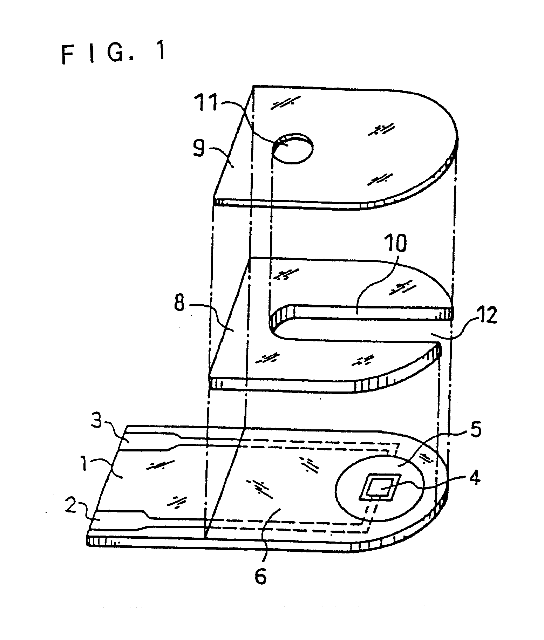

FIG. 1 is a perspective view of a disassembled biosensor according to an embodiment of the present invention excluding a reaction layer.

Reference numeral 1 indicates an electrically insulating base plate made of polyethylene terephthalate. On the base plate 1, leads 2 and 3 made of silver paste are formed by screen printing. Further, by the same printing method, are formed on the base plate 1 an electrode system including a working electrode 4 and a counter electrode 5 made of conductive carbon paste containing a resin binder, and an electrically insulating layer 6 made of electrically insulating paste. The electrically insulating layer 6 exposes a certain area of the working electrode 4 and the counter electrode 5 and partially covers the leads. In other words, the electrically insulating layer 6 defines the exposed area.

Other than silver and carbon, platinum, gold and palladium may be used as a material for the leads and the electrodes.

The biosensor is assembled from the insulating base plate 1 made of polyethylene terephthalate, a cover 9, and a spacer 8 sandwiched between the base plate 1 and the cover 9. These components are bonded in a positional relationship as indicated by dashed lines shown in FIG. 1 to form the biosensor. The spacer 8 is provided with a slit 10 for forming a sample solution supply pathway and the cover 9 includes an air aperture 11. With the base plate 1, the cover 9 and the spacer 8 bonded as shown in FIG. 1, a cavity is formed as a sample solution supply pathway between the base plate 1 and the cover 9. The cavity includes an opening end of the slit as a sample solution supply port and terminates at the air aperture 11. In this explanation, the cover 9 and the spacer 8 are combined as a covering member. However, a single member having a groove corresponding to the slit 10 of the spacer 8 may be used.

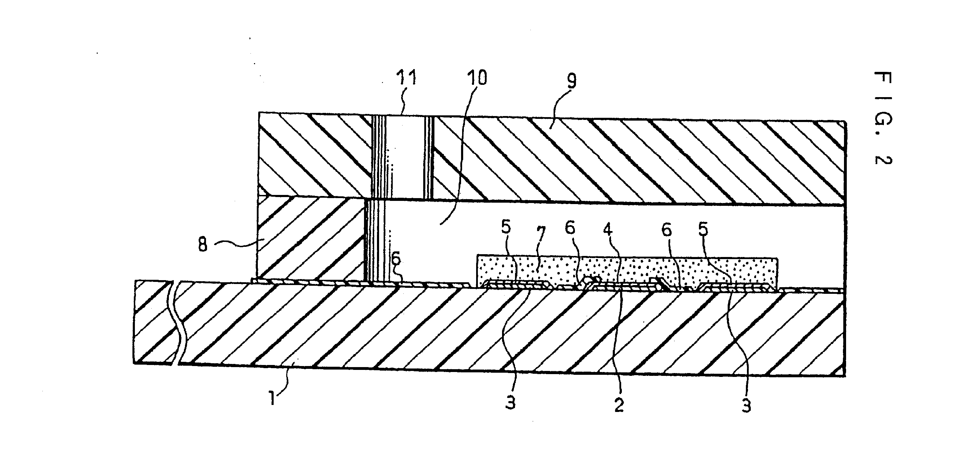

In FIG. 2, one or more reaction layers 7 containing at least an enzyme, an electron mediator and fine particles are formed on the insulating base plate 1 provided with the electrode system as shown in FIG. 1, i.e., in the slit of the covering member. The reaction layer is not necessarily formed on the electrode system as long as it is formed in a position exposed to the sample solution supply pathway and the sample solution flows over the electrode system while dissolving the reaction layer.

Hereinafter, the present invention is detailed by way of examples, but the invention is not limited thereto.

### Example 1

On the electrode system on the base plate 1 shown in FIG. 1, an aqueous solution containing fine particles of polystylene having an average diameter of 0.5 µm, potassium ferricyanide and glucose oxidase (EC1. 1. 3. 4, GOD) was dropped and dried in a warm-air dryer at 50°C for 10 minutes to form a reaction layer 7. The obtained reaction layer 7 contained 1 mg of the fine particles, 1 mg of potassium ferricyanide and 0.05 mg of GOD, per cm².

Then, the base plate was combined with the spacer and the cover to complete the sensor and sensor characteristic as a glucose sensor was evaluated.

On supplying a sample solution containing glucose to the reaction layer 7, glucose in the sample solution was oxidized by GOD and at the same time, the electron mediator in the reaction layer was reduced. After an elapse of a predetermined period of time, a voltage of +0.5 V with respect to the counter electrode 5 was applied to the working electrode 4 to oxidize the reduced electron mediator. Five seconds later, a current value between the working electrode and the counter electrode was measured. The current value was proportional to the concentration of the reduced electron mediator, i.e., the substrate concentration in the sample solution. The glucose concentration in the sample solution was obtained from the current value.

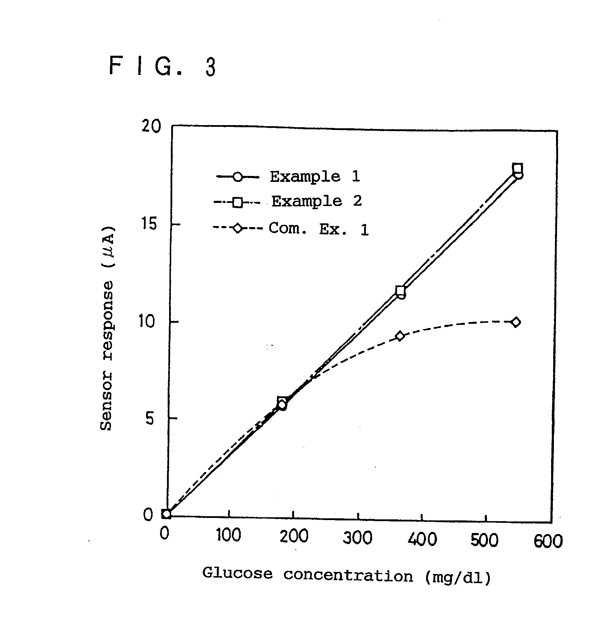

Samples containing glucose in concentrations of 0 mg/dl, 180 mg/dl, 360 mg/dl and 540 mg/dl were prepared and subjected to the measurement for 30 seconds, respectively, to measure the response current value of the sensor with respect to each sample. As a result, the response current value and the glucose concentration had a certain correlation and showed favorable linearity. Also in the measurement for 10 seconds, favorable linearity was obtained between the response current value and the glucose concentration. FIG. 3 shows the response characteristic obtained in the measurement for 10 seconds, together with the response characteristics obtained in Example 2 and Comparative Example 1.

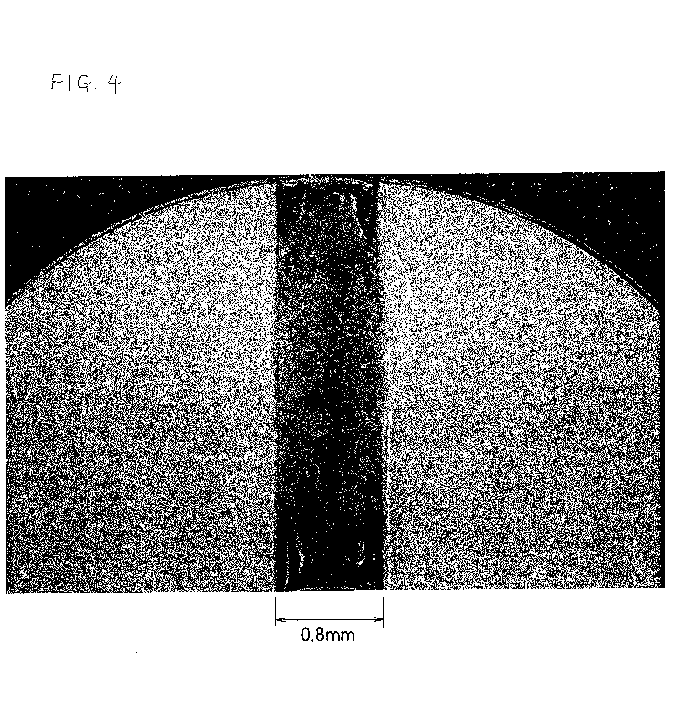

Further, with an optical microscope, the reaction layer 7 formed as described above was observed whether at least the surface was porous or not. FIG. 4 shows a photomicrograph taken under a magnification of 42.5 times. As seen in FIG. 4, the surface of the reaction layer 7 was porous.

The porosity of the reaction layer was obtained as a percentage of a volume of components in the reaction layer, which was obtained from the weight and specific gravity thereof, with respect to a volume of the reaction layer after drying. The porosity of the reaction layer was about 79.5 %.

### Example 2

An aqueous solution containing potassium ferricyanide was prepared, which was dropped and dried on the electrodes on the base plate 1 shown in FIG. 1 to form a first layer. The content of potassium ferricyanide in the first layer was 1 mg per cm².

Then, an aqueous solution containing GOD and fine polymer particles same as those of Example 1 was prepared, which was dropped on the first layer and dried in a warm-air dryer at 50°C for 10 minutes to form a second layer. The obtained second layer contained 1.0 mg of the fine polymer particles and 0.1 mg of GOD, per cm² of the second layer. The first and second layers were formed to function as the reaction layer 7.

In the same manner as Example 1, the response current value of the sensor with respect to a standardized glucose solution was measured. As a result, the response current value showed favorable linearity with respect to the glucose concentration in both of the measurements for 30 seconds and 10 seconds. The porosity of the reaction layer was about 79 %.

### Comparative Example 1

An aqueous solution containing potassium ferricyanide and GOD was dropped on the electrodes on the base plate 1 shown in FIG. 1 and dried in a warm-air dryer at 50°C for 10 minutes to form a reaction layer. The content of potassium ferricyanide in the reaction layer was 1 mg and the content of GOD was 0.05 mg, per cm².

In the same manner as Example 1, the response current value of the sensor with respect to a standardized glucose solution was measured. As a result, the response current value and the glucose concentration showed favorable linearity in the measurement for 30 seconds. However, the linearity was not obtained with respect to glucose of high concentration in the measurement for 10 seconds.

### Example 3

An aqueous solution containing fine particles of amorphous silicon having an average diameter of 0.3 µm, potassium ferricyanide and GOD was prepared. Using the solution, the reaction layer 7 was formed on the electrodes on the base plate 1 shown in FIG. 1 in the same manner as Example 1. The reaction layer 7 contained 1.5 mg of the fine particles, 1 mg of potassium ferricyanide and 0.05 mg of GOD, per cm².

In the same manner as Example 1, the response current value of the sensor with respect to a standardized glucose solution was measured. As a result, the response current value showed favorable linearity with respect to the glucose concentration in both of the measurements for 30 seconds and 10 seconds. The porosity of the reaction layer was about 74.5 %.

### Example 4

An aqueous solution containing potassium ferricyanide was prepared, which was dropped and dried on the electrodes on the base plate 1 shown in FIG. 1 to form a first layer. The content of potassium ferricyanide in the first layer was 1 mg per cm².

Then, an aqueous solution containing GOD and fine particles of amorphous silicon same as those in Example 3 was prepared, which was dropped on the first layer and dried in a warm-air dryer at 50°C for 10 minutes to form a second layer. In the obtained second layer, the content of the fine polymer particles was 1.5 mg and the GOD content was 0.1 mg, per cm². The first and second layers were formed to function as the reaction layer 7.

In the same manner as Example 1, the response current value of the sensor with respect to a standardized glucose solution was measured. As a result, the response current value showed favorable linearity with respect to the glucose concentration in both of the measurements for 30 seconds and 10 seconds. The porosity of the reaction layer was about 74 %.

### Comparative Example 2

An aqueous solution containing potassium ferricyanide was prepared, which was dropped and dried on the electrodes on the base plate 1 shown in FIG. 1 to form a first layer. The content of potassium ferricyanide in the first layer was 1 mg per cm².

Then, an aqueous solution containing GOD and fine particles of amorphous silicon having an average diameter of 1.5 µm was prepared. However, in this aqueous solution, the fine particles were precipitated immediately and hence the solution required constant stirring during a dropping step. This solution was dropped on the first layer and dried in a warm-air dryer at 50°C for 10 minutes to form a second layer. That is, the first and second layers were formed to function as the reaction layer 7. The content of the fine polymer particles in the thus formed second layer was 1.3 mg per cm² of the reaction layer. However, the content of the fine polymer particles was varied among sensors, which caused variations in GOD amount. The GOD amount was 0.1 mg on average per cm² of the reaction layer.

In the same manner as Example 1, the response current value of the sensor with respect to a standardized glucose solution was measured. The response current value was dependent on the glucose concentration in both of the measurements for 30 seconds and 10 seconds. However, when plural sensors were subjected to the measurement of the response current value with respect to the same standardized glucose solution, the response current values were varied in a wide range.

### Example 5

A first layer containing potassium ferricyanide was formed in the same manner as Example 2.

Then, an aqueous solution containing cholesterol esterase, cholesterol oxidase, fine particles of amorphous silica having an average diameter of 0.5 µm and Triton X-100 as a surfactant was prepared. The solution was dropped on the first layer and dried in a warm-air dryer at 50°C for 10 minutes to form a second layer. That is, the first and second layers were formed to function as the reaction layer 7. The second layer contained 0.10 mg of cholesterol oxidase and 0.05 mg of cholesterol esterase, per cm² of the reaction layer. The content of the fine particles was 1 mg and that of Triton X-100 was 0.4 mg, per cm² of the reaction layer.

A cholesterol sensor was fabricated in the same manner as the production of the glucose sensor and the response current value of the sensor with respect to a sample solution containing cholesterol was measured. As a result, the response current value showed favorable linearity with respect to the cholesterol concentration in the measurements for 3 minutes and 1 minute.

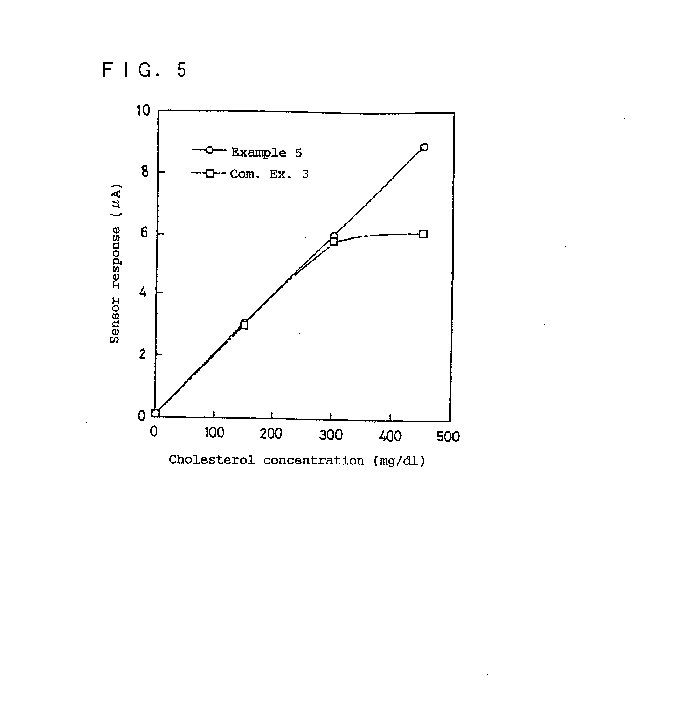

FIG. 5 shows the response characteristics of the sensors of this example and Comparative Example 3 in the measurement for 10 seconds. The porosity of the above reaction layer was about 74.5%.

### Comparative Example 3

On the electrode system on the base plate 1 shown in FIG. 1, an aqueous solution mixture of potassium ferricyanide, cholesterol oxidase and cholesterol esterase was dropped, which was dried in a warm-air dryer at 50°C for 10 minutes to form a reaction layer 7. The obtained reaction layer 7 contained 1 mg of potassium ferricyanide, 0.1 mg of cholesterol oxidase and 0.05 mg of cholesterol esterase, per cm² of the reaction layer 7.

In the same manner as Example 3, the response current value of the sensor was measured. As a result, favorable linearity was not obtained in both of the measurements for 1 minute and 3 minutes.

### Comparative Example 4

A first layer containing potassium ferricyanide was formed in the same manner as Example 3.

Then, an aqueous solution containing cholesterol esterase, cholesterol oxidase, fine particles of amorphous silicon having an average diameter of 1.5 µm and Triton X-100 as a surfactant was prepared. The solution was dropped on the first layer and dried in a warm-air dryer at 50°C for 10 minutes to form a second layer. That is, the first and second layers were formed to function as the reaction layer 7. In this solution, however, the fine particles were immediately precipitated, which required constant stirring during the dropping step. The second layer contained 0.10 mg of cholesterol oxidase and 0.05 mg of cholesterol esterase per cm² of the reaction layer. The content of the fine particles was 0.5 to 1.5 mg and that of Triton X-100 was 1.0 mg, per cm² of the reaction layer.

In the same manner as Example 3, the response current value of the sensor was measured. As a result, favorable response linearity was not obtained in both of the measurements for 1 minute and 3 minutes. The reason is presumably that the fine particles in the solution for forming the reaction layer were hard to disperse homogeneously and the fine particle amount in the reaction layer assembled in the sensor was not uniform, which caused considerable variations in response value of the sensor in evaluating the response characteristics.

### Example 6

On a platinum electrode system formed on the base plate 1 shown in FIG. 1, an aqueous solution containing glucose oxidase (GOD) and fine particles (beads) of polystylene having an average diameter of 0.8 µm was dropped to form a reaction layer 7. The obtained reaction layer 7 contained 0.03 mg of GOD and 1.5 mg of the fine particles, per cm² of the reaction layer.

On the electrode system, a sample solution containing glucose was supplied. Glucose in the sample solution was oxidized by GOD to generate hydrogen peroxide. Ten seconds after the sample solution was supplied, a voltage of +1.0V with respect to the counter electrode 5 was applied to the working electrode 4 to measure a current value after 5 seconds. This current value was proportional to the substrate concentration in the sample solution, from which the glucose concentration in the sample solution was obtained.

Samples containing glucose in concentrations of 0 mg/dl, 180 mg/dl, 360 mg/dl and 540 mg/dl were prepared and subjected to the measurement for 30 seconds, respectively, to measure the response current value of the sensor with respect to each solution. As a result, the response current value and the glucose concentration had a certain correlation and showed favorable linearity. Further, also in the measurement for 10 seconds, favorable linearity was obtained between the response current value and the glucose concentration. The porosity of the reaction layer was about 84.7 %.

In the case where the fine particles were not mixed in the reaction layer, the response linearity was lost with respect to the substrate of high concentration. The reason for the above is supposedly that the reaction layer without the fine particles is dried to have a dense structure, and hence only the surface is dissolved to cause reaction with the sample solution supplied to the sensor.

As compared with the reaction layer without the fine particles, in the reaction layer formed by mixing the fine particles the enzyme and the electron mediator are dried into a thin film on the fine particle surface. Since the sample solution permeates into the reaction layer very rapidly through the voids in the fine particles, the reagent including the enzyme dissolves smoothly and the enzyme reaction proceeds promptly. As a result, the measurement with the sensor can be carried out in a reduced period.

In this case, if the fine particles contained in the reaction layer have an average diameter smaller than 0.1 µm, the structure of the reaction layer containing the fine particles becomes very minuscule and voids are not generated in the fine particles. Therefore, the reaction layer does not dissolve smoothly into the sample solution. On the other hand, if the average diameter of the fine particles is 1 µm or more, the fine particles may precipitate in the solution for forming the reaction layer or coagulate on or near the solution surface and hence the production process is complicated.

From the above, the effective size of the fine particles is in the range of not smaller than 0.1 µm and not larger than 1 µm. With the reaction layer containing the fine particles so defined, the response characteristic of the sensor becomes very favorable even in measuring a sample solution containing various components such as hemocytes and protein, e.g., blood.

In the above-described examples, explanation is given in terms of the glucose sensor and the cholesterol sensor. However, the present invention is also applicable to other sensors such as a glucose or cholesterol sensor using other enzymes, a lactic acid sensor, a fructose sensor, a sucrose sensor, an alcohol sensor, an ascorbic acid sensor and the like.

As described above, according to the present invention, there is provided a biosensor capable of determining the quantity of a substrate contained in a sample, e.g., biological samples such as blood and urea and materials and products in food industries, in a highly accurate, speedy and easy manner.

Although the present invention has been described in terms of the presently preferred embodiments, it is to be understood that such disclosure is not to be interpreted as limiting. Various alterations and modifications will no doubt become apparent to those skilled in the art to which the present invention pertains, after having read the above disclosure. Accordingly, it is intended that the appended claims be interpreted as covering all alterations and modifications as fall within the true spirit and scope of the invention.

## Claims

1. A biosensor comprising an electrically insulating base plate, an electrode system including a working electrode and a counter electrode formed on the base plate, and a reaction layer formed on or in the vicinity of the electrode system, at least a surface of the reaction layer being porous.

2. A biosensor according to claim 1, wherein the reaction layer contains at least an enzyme and an aggregate of fins particles having an average diameter of between 0.1 µm and 1 µm.

3. A biosensor according to claim 2, wherein the enzyme is one or a combination of two or more chosen from glucose oxidase, glucose dehydrogenase, lactate oxygenase, lactate dehydrogenase, fructose oxidase, cholesterol oxidase, cholesterol dehydrogenase, cholesterol esterase, mutarotase, invertase, ascorbate oxidase and alcohol oxidase.

4. A biosensor according to claim 2 or claim 3, wherein the fine particles are made of a material selected from the group consisting of a polymer compound, ceramic, glass, diamond and carbon.

5. A biosensor according to any one of the preceding claims wherein the reaction layer further contains an electron mediator.

6. A biosensor according to any one of claims 1 to 5, wherein a layer containing an electron mediator is formed in the vicinity of the reaction layer.

7. A biosensor according to claim 6, wherein the reaction layer contains cholesterol esterase, a surfactant and at least one of cholesterol oxidase and cholesterol dehydrogenase, and a layer containing an electron mediator is formed in the vicinity of the reaction layer.

8. A biosensor according to any one of claims 5 to 7 wherein the electron mediator is one or a combination of two or more chosen from ferricyanide and salts thereof; p-benzoquinone and derivatives thereof; phenazine methosulfate; methylene blue; and ferrocene and derivatives thereof.

9. A biosensor according to any one of the preceding claims capable of determining the quantity of glucose, cholesterol, lactic acid, fructose, sucrose, alcohol or ascorbic acid in a sample.

10. A method for producing a biosensor according to any one of the preceding claims comprising:
(i) dispersing fine particles in a solution for forming a reaction layer;
(ii) spreading the resulting solution on or in the vicinity of an electrode system on a base plate; and
(iii) drying said solution.
